# FASCICULE DE BREVET EUROPEEN

(11) **EP 1 367 952 B1**
(45) Date de publication et mention de la délivrance du brevet: **28.03.2007**
(21) Numéro de dépôt: 01993430.6
(22) Date de dépôt: 05.11.2001
(51) Int. Cl.: A61B 17/70

(54) **MATERIEL D'ARTHRODESE VERTEBRALE**
AUSRÜSTUNG FÜR DIE WIRBELARTHRODESE
VERTEBRAL ARTHRODESIS EQUIPMENT

(30) Priorité: 07.11.2000 FR 0014274
(43) Date de publication de la demande: 10.12.2003
(73) Titulaire: Médicréa Technologies, 17000 La Rochelle (FR)
(72) Inventeur: TAYLOR, Jean, F-06400 CANNES (FR); VILLARET, Bernard, F-17220 CROIX CHAPEAU (FR); PARISINI, Patrizio, 40123 BOLOGNE (IT); CLEMENT, Jean-Luc, F-06480 LA COLLE SUR LOUP (FR); ADAM, Yves, F-14280 AUTHIE (FR)
(74) Mandataire: Jeannet, Olivier
(86) Numéro de dépôt international: PCT/FR2001/003411
(87) Numéro de publication internationale: WO 2002/038060

(56) Documents cités:
- WO-A-98/55038
- NL-C- 1 011 260

## Description

La présente invention concerne un matériel d'arthrodèse vertébrale.

Un tel matériel comprend généralement deux tiges d'étayage, destinées à être disposées parallèlement l'une à l'autre de part et d'autre des vertèbres à traiter, et des organes d'ancrage de ces tiges aux vertèbres, tels que des crochets ou des vis pédiculaires. Ce matériel peut éventuellement comprendre des traverses qui relient transversalement ces tiges de proche en proche pour les maintenir l'une par rapport à l'autre.

Pour assembler une tige d'étayage à un organe d'ancrage, il a notamment été conçu d'aménager deux parois en vis-à-vis sur la partie proximale de l'organe d'ancrage, ces parois délimitant entre elles un logement de réception de la tige et présentant des moyens, tels qu'un filetage ou un taraudage, pour recevoir un écrou ou un bouchon fileté de blocage de la tige. Ces parois confèrent à cette partie proximale une forme en "diapason" ou en "tulipe", ces appellations étant devenues courantes pour désigner ce type de montage.

Un crochet existant sur ce type de matériel est destiné à être engagé autour du pédicule et présente à cet effet une lame recourbée conformée en conséquence. Les possibilités d'utilisation de ce crochet sont toutefois limitées à une prise d'appui pédiculaire, ce qui ne permet pas de s'adapter aux différentes situations pouvant se présenter. En effet, dans certains cas, en particulier selon la position et l'orientation de la tige d'étayage au niveau de ce crochet ou selon la qualité de l'os de la vertèbre, il peut être préférable de rechercher une autre zone d'appui.

Le crochet existant peut également susciter, dans des cas limites, des incertitudes quant à la parfaite résistance du montage qu'il permet.

La présente invention vise, à titre principal, à remédier à ces inconvénients essentiels.

Par ailleurs, les crochets en "diapason" ("tulipe") existants ont pour inconvénient important d'obliger parfois à des efforts importants pour amener les tiges à l'intérieur des logements de réception de ces tiges. Lorsque les pédicules présentent des petites dimensions, comme cela est le cas des vertèbres dorsales, en particulier des vertèbres D1 à D4, des risques de rupture pédiculaire existent et il est nécessaire de recourir plutôt à des crochets qu'à des vis. Compte tenu des limitations précitées, les crochets existants ne s'avèrent pas parfaitement adaptés à toutes les situations pouvant se présenter.

En outre, les crochets existants ont pour inconvénient d'avoir une hauteur importante du fait de la hauteur desdites parois délimitant les logements de réception d'une tige d'étayage. Cette hauteur n'est pas particulièrement gênante lorsque les tissus ont une épaisseur importante, comme cela est le cas au niveau des vertèbres lombaires, mais est par contre gênante lorsque ces tissus sont de relativement faible épaisseur, comme cela est le cas au niveau des vertèbres dorsales, le matériel étant alors sensible sous la peau.

La présente invention vise également à remédier à cet inconvénient.

Le matériel qu'elle concerne comprend, de manière connue en soi, au moins une tige d'étayage et au moins un ensemble d'ancrage de la tige d'étayage à une vertèbre ; chaque ensemble d'ancrage comprend une embase solidaire d'un crochet et des moyens pour l'assemblage de ladite tige d'étayage à l'ensemble d'ancrage ; le crochet présente une partie de base courbe, par laquelle il est raccordé à l'embase, et une partie sensiblement plane, s'étendant dans un plan parallèle, ou formant un angle faible, inférieur à 15 degrés, avec cette embase.

Un tel matériel est divulgué dans la demande de brevet internationale WO 98/55038, et le préambule de la revendication 1 est basé sur ce matériel.

Selon l'invention,
- l'embase présente un trou qui la traverse de part en part, aménagé selon une direction parallèle, ou formant un angle faible tel que précité, avec la direction longitudinale de ladite partie plane du crochet; ce trou débouche, du côté de la base du crochet, par un orifice de section circulaire et présente, en dehors de cet orifice, une section transversale oblongue, la longueur de cette section oblongue s'étendant dans un plan parallèle, ou formant un angle faible tel que précité, avec le plan dans lequel s'étend ladite partie plane ; et
- l'ensemble d'ancrage comprend une pièce en forme de crochet, dont la partie correspondant à la base du crochet est reliée à une tige filetée, cette tige pouvant être engagée dans ledit trou avec possibilité de pivotement selon son axe et de débattement dans ledit plan, et pouvant recevoir un écrou sur son extrémité débouchant au travers dudit orifice, cet écrou permettant, lorsqu'il est vissé, de rapprocher le crochet que forme ladite pièce du crochet solidaire de l'embase.

Chaque ensemble comprend ainsi un premier crochet solidaire de ladite embase et un deuxième crochet, antagoniste au premier crochet, pouvant être placé dans différentes positions par rapport au premier crochet, à savoir selon plusieurs positions de pivotement autour de l'axe de la tige filetée et selon plusieurs positions angulaires par rapport à l'embase, rendues possibles par le débattement de cette tige dans ledit trou.

Ces deux crochets permettent d'enserrer entre eux deux zones vertébrales d'appui, dont au moins une peut être choisie de manière à correspondre au mieux à l'ancrage à réaliser, en fonction de la position ou de l'orientation de la tige d'étayage et/ou en fonction de la qualité de l'os. L'ensemble selon l'invention permet ainsi notamment des prises d'appui pédiculo-lamaire, lamaire-transversaire, pédiculo-transversaire ou lamaire-lamaire.

Avantageusement, les moyens pour l'assemblage d'une tige d'étayage à un ensemble d'ancrage tel que précité comprennent :
- un bossage solidaire de l'embase, délimitant intérieurement une cavité de forme au moins partiellement sphérique et délimitant extérieurement une portion de paroi sphérique ; les centres générant la ou les zones sphériques de la cavité et ladite portion de paroi sphérique sont décalés l'un par rapport à l'autre selon une direction perpendiculaire à l'embase ;
- une tige filetée comprenant, à une extrémité, une base renflée destinée à être engagée et à être retenue dans ladite cavité de l'embase, cette base renflée présentant une forme au moins partiellement sphérique, propre à coopérer avec la ou les zones sphériques de la cavité de manière à permettre le pivotement ou l'articulation de la tige filetée par rapport à l'embase ;
- un étrier présentant une partie arrondie propre à recevoir une tige d'étayage, deux ailes superposées percées de trous pour permettre l'engagement de l'étrier sur ladite tige filetée, et une face en portion de sphère creuse, cette face étant destinée à venir porter contre ladite portion de paroi sphérique lorsque l'étrier est engagé sur ladite tige filetée, et
- un écrou pouvant être vissé sur la tige filetée de manière à permettre le serrage de l'étrier entre lui et ladite portion de paroi sphérique.

Grâce à la cavité et à la base renflée, la tige filetée peut être orientée en direction de l'étrier, lui-même préalablement engagé autour de la tige d'étayage. Cette orientation permet de faciliter l'engagement des oeillets de l'étrier sur cette tige filetée.

L'écrou peut alors être engagé sur la tige filetée et être vissé, ce qui a pour effet d'amener ladite face concave de l'étrier au contact de ladite portion de paroi sphérique du bossage puis, une fois ce contact intervenu, et compte tenu du décalage des deux centres précités, d'amener progressivement, au fur et à mesure du serrage de l'écrou, ladite tige filetée dans une direction sensiblement perpendiculaire à l'embase.

Ces moyens d'assemblage facilitent ainsi la mise en place du matériel et permettent une venue progressive de la tige d'étayage en position par rapport aux ensembles d'ancrage que comprend ce matériel, préalablement mis en place sur les vertèbres.

Pour sa bonne compréhension, l'invention est à nouveau décrite ci-dessous en référence au dessin schématique annexé représentant, à titre d'exemple non limitatif, une forme de réalisation préférée du matériel qu'elle concerne.
La figure 1 est une vue en perspective, avant montage, d'un ensemble d'ancrage qu'il comprend ;
les figures 2 à 4 sont des vues respectivement de côté, de face et de dessus d'une embase et d'un crochet qu'il comprend, et
la figure 5 est une vue partielle, à échelle agrandie et après montage, de l'ensemble d'ancrage, en coupe selon la ligne V-V de la figure 1.

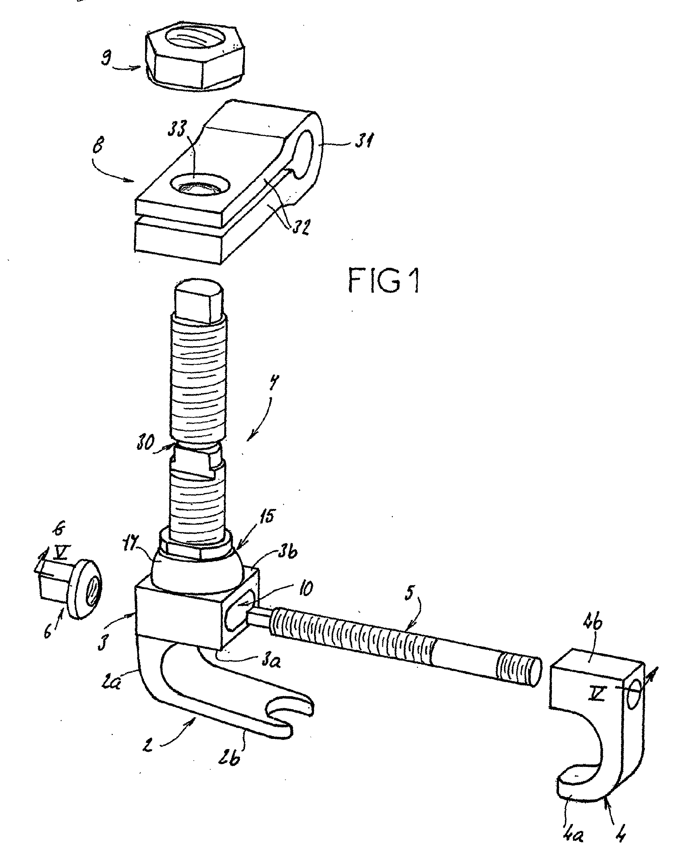

La figure 1 représente un ensemble d'ancrage 1 faisant partie d'un matériel d'arthrodèse vertébrale.

Ce matériel comprend deux tiges d'étayage, destinées à être disposées parallèlement l'une à l'autre de part et d'autre des vertèbres, et des organes d'ancrage de ces tiges aux vertèbres. Ces organes d'ancrage peuvent être des vis pédiculaires et/ou un ou plusieurs ensembles d'ancrage 1. Le matériel peut comprendre en outre des traverses destinées à relier transversalement les tiges d'étayage de proche en proche pour les maintenir l'une par rapport à l'autre.

Ces tiges, vis pédiculaires et traverses sont bien connues en elles-mêmes et ne sont donc pas particulièrement décrites.

Comme le montre la figure 1, l'ensemble d'ancrage 1 comprend un crochet 2 solidaire d'une embase parallélépipédique 3, une pièce 4 en forme de crochet reliée à une tige filetée 5, un écrou 6 pouvant être vissé sur cette tige filetée 5, une tige filetée 7, un étrier 8 et un écrou 9.

Le crochet 2 présente une partie de base courbe 2a, par laquelle il est raccordé à l'embase 3, et une partie sensiblement plane 2b, formant un angle de huit degrés avec la face 3a de l'embase à laquelle le crochet 2 est raccordé.

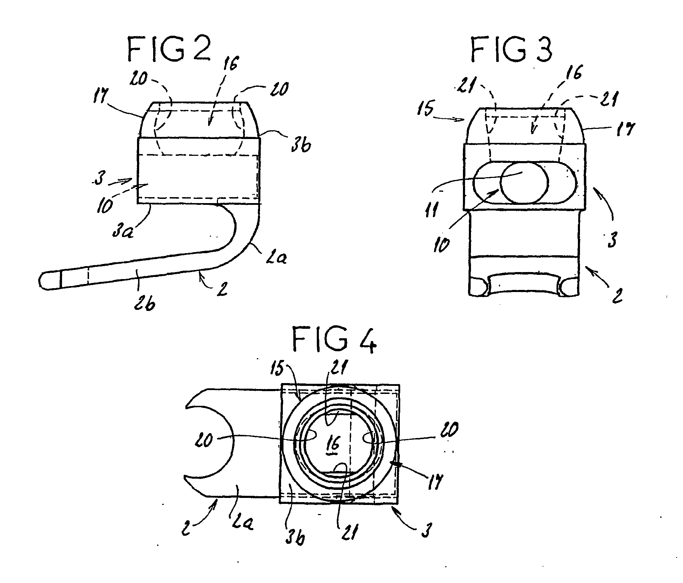

L'embase 3 présente un trou 10 qui la traverse de part en part. Ainsi que cela apparaît plus particulièrement sur les figures 3 et 4, ce trou 10 est aménagé globalement selon une direction parallèle à la face 3a de l'embase et selon la direction longitudinale de la partie plane 2b. Ce trou 10 débouche dans deux faces latérales opposées de l'embase 3 ; du côté de la partie de base 2a, ce trou 10 débouche par un orifice 11 de forme circulaire, ayant une section légèrement supérieure à la section transversale de la tige 5 ; en dehors de cet orifice 11, le trou 10 a une section transversale oblongue, la longueur de cette section oblongue s'étendant parallèlement à la face 3a.

L'embase 3 présente également un bossage 15 faisant saillie de sa face principale 3b opposée à la face 3a. Ce bossage 15 délimite intérieurement une cavité 16 et présente extérieurement une face 17 en portion de paroi sphérique.

Il apparaît sur les figures 3 et 4 que la cavité 16 est délimitée par deux zones 20 diamétralement opposées, en forme de sphère creuse et générées par un même rayon et un même centre, et par deux méplats 21, parallèles aux faces latérales de l'embase 3 dans lesquelles le trou 10 ne débouche pas.

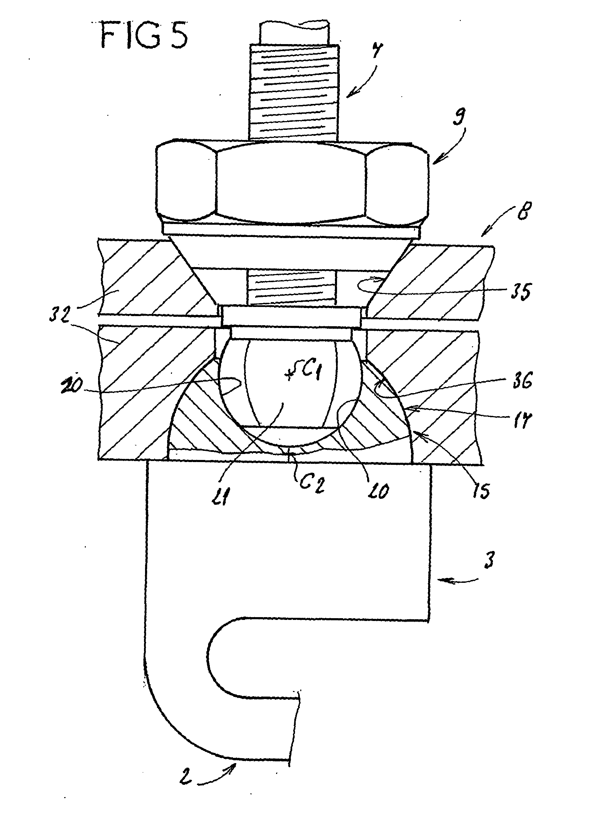

La figure 5 montre que les centres C1, C2 générant la ou les zones 20 et la portion de sphère que forme la face 17 sont décalés l'un par rapport à l'autre selon une direction perpendiculaire auxdites faces principales 3a, 3b de l'embase 3.

La pièce 4 présente une partie 4a recourbée en forme de crochet et une partie de base 4b. La partie de base 4b est reliée à la tige filetée 5.

Cette tige 5 présente un diamètre tel qu'elle peut être engagée dans le trou 10 avec possibilité de pivotement selon son axe et de débattement dans ce trou 10.

Sur son extrémité débouchant au travers de l'orifice 11, la tige 5 peut recevoir l'écrou 6. Ce dernier, lorsqu'il est vissé, permet ainsi de rapprocher le crochet 2 du crochet que forme la partie 4a de la pièce 4.

La tige 7 comprend, à une extrémité, une base renflée présentant deux zones sphériques et deux méplats ayant une forme qui correspond précisément aux zones 20 et méplats 21. Cette base est engagée dans la cavité 16 au moment de la fabrication de l'ensemble 1 puis la paroi latérale du bossage 15 est sertie sur cette base de manière à assurer la rétention de la base dans la cavité 16, avec possibilité de pivotement de la tige 7 par rapport à l'embase 3, par glissement des zones sphériques les unes contre les autres.

La tige 7 présente également une portion amincie 30, permettant sa section après montage définitif du matériel.

L'étrier 8 présente une partie arrondie 31 délimitant un logement propre à recevoir la tige d'étayage, et deux ailes latérales 32 dans lesquelles sont aménagés deux trous 33 superposés. Ces trous 33 permettent l'engagement de l'étrier 8 sur la tige 7 de manière à relier la tige d'étayage à l'ensemble 1.

Comme le montre la figure 5, le trou 33 supérieur est délimité par une face conique 35 et le trou 33 inférieur est délimité par une face 36 en portion de sphère creuse de même rayon que la face 17.

L'ensemble 1 comprend ainsi un premier crochet 2 solidaire de l'embase 3 et un deuxième crochet 4b, antagoniste au premier crochet 2, pouvant être placé dans différentes positions par rapport au premier crochet 2, à savoir selon plusieurs positions de pivotement autour de l'axe de la tige filetée 5 et selon plusieurs positions angulaires par rapport à l'embase 3, rendues possibles par le débattement de cette tige 5 dans le trou 10. L'ensemble selon l'invention permet ainsi notamment des prises d'appui pédiculo-lamaire, lamaire-transversaire, pédiculo-transversaire ou lamaire-lamaire.

Par ailleurs, grâce à la cavité 16 et à la base renflée, la tige 7 peut, au moment de la mise en place du matériel, être orientée en direction de l'étrier 8 correspondant, préalablement engagé autour de la tige d'étayage. Cette orientation permet de faciliter l'engagement des trous 33 sur cette tige 7. L'écrou 9 peut alors être engagé sur la tige 7 et être vissé, ce qui a pour effet d'amener la face concave 36 au contact de la face 17 puis, une fois ce contact intervenu, et compte tenu du décalage des deux centres C1, C2 précité, d'amener progressivement, au fur et à mesure du serrage de l'écrou 9, la tige 7 dans une direction sensiblement perpendiculaire à l'embase 3.

Ces moyens d'assemblage facilitent ainsi la mise en place du matériel et permettent une venue progressive de la tige d'étayage en position par rapport à ou aux ensembles 1 que comprend ce matériel.

Ainsi qu'il apparaît de ce qui précède, l'invention fournit un matériel d'arthrodèse vertébrale présentant les avantages importants précités par rapport aux matériels homologues de la technique antérieure.

Il va de soi que l'invention n'est pas limitée à la forme de réalisation décrite ci-dessus à titre d'exemple mais qu'elle en embrasse au contraire toutes les variantes de réalisation couvertes par les revendications ci-annexées.

## Revendications

1. - Matériel d'arthrodèse vertébrale comprenant au moins une tige d'étayage et au moins un ensemble d'ancrage (1) de la tige d'étayage à une vertèbre ; chaque ensemble d'ancrage (1) comprend une embase (3) solidaire d'un crochet (2) qui présente une partie de base (2a) courbe, par laquelle il est raccordé à l'embase (3), et une partie sensiblement plane (2b), s'étendant dans un plan parallèle, ou formant un angle faible, inférieur à 15 degrés, avec cette embase (3) ; des moyens pour l'assemblage de ladite tige d'étayage à l'ensemble d'ancrage (1) ; et une pièce (4) en forme de crochet qui peut être rapprochée du crochet (2) solidaire de l'embase (3),
matériel **caractérisé en ce que** :
- l'embase (3) présente un trou (10) qui la traverse de part en part, aménagé selon une direction parallèle, ou formant un angle faible tel que précité, avec la direction longitudinale de ladite partie plane (2b) du crochet (2) ; ce trou (10) débouche, du côté de la base du crochet (2), par un orifice (11) de section circulaire et présente, en dehors de cet orifice (11), une section transversale oblongue, la longueur de cette section oblongue s'étendant dans un plan parallèle, ou formant un angle faible tel que précité, avec le plan dans lequel s'étend ladite partie plane (2b) ; et
- la partie (4b) correspondant à la base du crochet de la pièce (4) en forme de crochet est reliée à une tige filetée (5), cette tige (5) pouvant être engagée dans ledit trou (10) avec possibilité de pivotement selon son axe et de débattement dans ledit plan, et pouvant recevoir un écrou (6) sur son extrémité débouchant au travers dudit orifice (11), cet écrou (6) permettant, lorsqu'il est vissé, le rapprochement du crochet que forme ladite pièce (4) du crochet (2) solidaire de l'embase (3).

2. - Matériel selon la revendication 1, **caractérisé en ce que** les moyens pour l'assemblage d'une tige d'étayage à un ensemble d'ancrage (1) tel que précité comprennent :
- un bossage (15) solidaire de l'embase (3), délimitant intérieurement une cavité (16) de forme au moins partiellement sphérique et délimitant extérieurement une portion de paroi sphérique (17); les centres (C1, C2) générant la ou les zones sphériques (20) de la cavité (16) et ladite portion de paroi sphérique (17) sont décalés l'un par rapport à l'autre selon une direction perpendiculaire à l'embase (3) ;
- une tige filetée (7) comprenant, à une extrémité, une base renflée destinée à être engagée et à être retenue dans ladite cavité (16) de l'embase (3), cette base renflée présentant une forme au moins partiellement sphérique, propre à coopérer avec la ou les zones sphériques (20) de la cavité (16) de manière à permettre le pivotement ou l'articulation de la tige filetée (7) par rapport à l'embase (3) ;
- un étrier (8) présentant une partie arrondie (31) propre à recevoir une tige d'étayage, deux ailes superposées (32) percées de trous (33) pour permettre l'engagement de l'étrier (8) sur ladite tige filetée (7), et une face (36) en portion de sphère creuse, cette face (36) étant destinée à venir porter contre ladite portion de paroi sphérique (17) lorsque l'étrier (8) est engagé sur ladite tige filetée (7), et
- un écrou (9) pouvant être vissé sur la tige filetée (7) de manière à permettre le serrage de l'étrier (8) entre lui et ladite portion de paroi sphérique (17).

3. - Matériel selon la revendication 2, **caractérisé en ce que**:
- la cavité (16) est délimitée par deux zones (20) diamétralement opposées, en forme de sphère creuse et générées par un même rayon et un même centre, et par deux méplats (21), parallèles aux faces latérales de l'embase (3) dans lesquelles le trou (10) ne débouche pas ; et **en ce que**
- ladite base renflée présente deux zones sphériques et deux méplats ayant une forme qui correspond précisément aux zones (20) et méplats (21) de l'embase (3).

4. - Matériel selon la revendication 2 ou la revendication 3, **caractérisé en ce que** la tige filetée (7) présente une portion amincie (30), permettant sa section après montage définitif du matériel.

## Claims

1. A vertebral arthrodesis equipment including at least a shoring rod and at least an anchoring assembly (1) of the shoring rod to a vertebra ; each anchoring assembly (1) comprises a base (3) integral with a hook (2) which has a curved base part (2a), by means of which it is attached to the base (3), and a substantially planar part (2b) extending in a parallel plane, or forming a slight angle, less than 15 degrees, with this base (3) ; means for attaching said shoring rod to the anchoring assembly (1) ; and a hook-shaped component (4) which can be brought closer the hook (2) integral with the base (3);
which equipment is **characterized in that**:
- the base (3) has a hole (10) which runs right through it, extending in a parallel direction or forming a slight angle as described in the foregoing, with the longitudinal direction of said planar part (2b) of the hook (2) ; this hole (10) opens towards the hook base (2) in the form of an opening (11) having a circular section and, apart from this opening (11) has an oblong cross-section, the length of which oblong cross-section extending on a parallel plane or forming a slight angle, as described in the foregoing, with the plane in which said planar part (2b) extends ; and
- the part (4b) corresponding to the base of the hook of the hook-shaped component (4) whereof is linked to a threaded rod (5), said rod (5) capable of being engaged in said hole (10) with the possibility of pivoting about an axis and of displacement in said plane, and capable of receiving a nut (6) at the end that protrudes through said opening (11), and which nut (6), when threaded bringing closer the hook formed by the component (4) to be drawn towards the hook (2) that is integral with the base (3).

2. The equipment according to claim 1, **characterized in that** the means for attaching a shoring rod to an anchoring assembly (1) as described include:
- a boss (15) integral with the base (3), defining an interior cavity (16) that is at least partly spherical in shape, and defining on the outside a portion of a spherical wall (17); the centres (C1, C2) that define the one or more spherical zones (20) of the cavity (16) and the said portion of the spherical wall (17) are offset with respect to each other in a direction perpendicular to the base (3);
- a threaded rod (7) including at one end thereof a convex base designed to be engaged and retained in said cavity (16) of the base (3), this convex base being at least partly spherical in shape, so that it cooperates with the one or more spherical zones (20) of the cavity (16) so as to allow pivoting or articulation of the threaded rod (7) relative to the base (3);
- a stirrup piece (8) having a rounded part (31) suitable for receiving a shoring rod, two superposed wings (32) perforated with holes (33) to allow the stirrup piece (8) to engage with said threaded rod (7), and a face (36) in the shape of a portion of hollow sphere, which face (36) being designed to abut said portion of the spherical wall (17) when the stirrup piece (8) is engaged on said threaded rod (7), and
- a nut (9) that may be screwed onto the threaded rod (7) so as to enable the stirrup piece (8) to be tightened between the nut and said portion of the spherical wall (17).

3. The equipment according to claim 2, **characterised in that**:
- the cavity (16) is defined by two diametrically opposed zones (20) in the form of a hollow sphere and formed on the same radius and the same centre, and by two flattened surfaces (21), parallel to the lateral faces of base (3) in which hole 10 does not have an opening ; and **in that**
- the said convex base has two spherical zones and two flattened surfaces to yield a shape corresponding precisely with zones (20) and flattened surfaces (21) of base (3).

4. The equipment according to claim 2 or claim 3, **characterised in that** threaded rod (7) has a reduced section (30) that allows section thereof after the equipment has been finally assembled.

## Patentansprüche

1. Wirbelarthrodesematerial mit mindestens einem Stützschaft und einer Verankerungseinheit (1) des Stützschafts an einem Wirbel, wobei jede Verankerungseinheit (1) einen Sockel (3) der fest mit einem Haken (2) verbunden ist, der einen gebogenen Basisteil (2a) aufweist, über welchen er mit dem Sockel (3) verbunden ist, und einen im Wesentlichen ebenen Teil (2b), der sich in einer Ebene parallel oder einen kleinen Winkel, kleiner als 15 Grad zu dieser Basis bildend erstreckt, sowie einen Teil (4) in Hakenform, der dem Haken (2), der fest mit dem Sockel (3) verbunden ist, angenähert werden kann,
Material **dadurch gekennzeichnet,**
- **dass** der Sockel (3) ein Loch (10) aufweist, das ihn von einer Seite zur anderen durchquert, eingerichtet entlang einer parallelen Richtung oder einen wie oben genannten kleinen Winkel, zu der Längsrichtung des ebenen Teils (2b) des Hakens (2) bildend, wobei dieses Loch (10) auf der Seite der Basis des Hakens (2) über eine Öffnung (11) mit kreisförmigem Querschnitt mündet und außerhalb dieser Öffnung (11) einen länglichen Querschnitt aufweist, wobei sich die Länge dieses länglichen Querschnitts in einer Ebene erstreckt, die parallel ist oder einen kleinen Winkel wie oben genannt mit der Ebene bildet, in der sich der ebene Teil (2b) erstreckt, und
- **dass** der Teil (4b), der der Basis der Hakens des Teils (4) in Hakenform entspricht, mit einem Gewindeschaft (5) verbunden ist, wobei dieser Gewindeschaft (5) in das Loch (10) mit der Möglichkeit des Schwenkens um seine Achse und Ausschlagens in der Ebene eingefügt werden kann und eine Mutter (6) an seinem Ende, das durch die Öffnung (11) mündet, aufnehmen kann, wobei diese Mutter (6), wenn sie geschraubt wird, das Annähern des Hakens erlaubt, den der Teil (4) des Hakens (2), der mit dem Sockel (3) fest verbunden ist, bildet.

2. Material nach Anspruch 1, **dadurch gekennzeichnet, dass** die Mittel zum Zusammenbauen eines Stützschafts mit einer Verankerungseinheit (1) wie oben genannt, Folgendes aufweisen:
- einen Höcker (15), der mit dem Sockel (3) fest verbunden ist, der innen einen Hohlraum (16) mit zumindest teilweise sphärischer Form bildet, und außen einen sphärischen Wandabschnitt (17) abgrenzt, wobei die Mitten (C1, C2), die die sphärische Zone(n) (20) des Hohlraums (16) erzeugen und der sphärische Wandabschnitt (17) voneinander entlang einer Richtung senkrecht zu dem Sockel (3) versetzt sind,
- einen Gewindestift (7), der an einem Ende eine gewölbte Basis aufweist, die dazu bestimmt ist, eingefügt und in dem Hohlraum (16) des Sockels (3) zurückgehalten zu werden, wobei diese gewölbte Basis eine zumindest teilweise sphärische Form aufweist, die mit der/den sphärischen Zone(n) (20) des Hohlraums (16) so zusammenwirken kann, dass das Schwenken oder Anlenken des Gewindestifts (7) in Bezug auf die Basis (3) erlaubt wird,
- einen Bügel (8), der einen gerundeten Teil (31) aufweist, der einen Stützschaft aufnehmen kann, zwei übereinander angeordnete Flügel (32), die mit Löchern (33) durchbohrt sind, um das Einfügen des Bügels (8) auf dem Gewindestift (7) zu erlauben, und eine Seite (36) in Hohlkugelabschnitt, wobei diese Seite (36) dazu bestimmt ist, gegen den sphärischen Wandabschnitt (17) zum Aufliegen zu kommen, wenn der Bügel (8) auf dem Gewindestift (7) eingefügt ist, und
- eine Mutter (9), die so auf den Gewindestift (7) geschraubt werden kann, dass das Festziehen des Bügels (8) zwischen ihm und dem sphärischen Wandabschnitt (17) erlaubt wird.

3. Material nach Anspruch 2, **dadurch gekennzeichnet,**
- **dass** der Hohlraum (16) von zwei Zonen (20) die einander diametral entgegengesetzt sind, in Hohlkugelform und die von einem gleichen Radius und einer gleichen Mitte erzeugt werden, abgegrenzt ist, und durch zwei Flachteile (21), die zu den seitlichen Seiten der Basis (3), in welche das Loch (10) nicht mündet, parallel sind, und dadurch,
- **dass** die gewölbte Basis zwei sphärische Zonen und zwei Flachteile aufweist, die eine Form haben, die genau den Zonen (20) und Flachteilen (21) des Sockels (3) entspricht.

4. Material nach Anspruch 2 oder 3, **dadurch gekennzeichnet, dass** der Gewindestift (7) einen verdünnten Abschnitt (30) aufweist, der sein Abtrennen nach dem endgültigen Montieren des Materials erlaubt.
